# EUROPEAN PATENT APPLICATION

(11) **EP 4 230 180 A1**
(43) Date of publication of application: **23.08.2023**
(21) Application number: 21880064.7
(22) Date of filing: 11.10.2021
(51) Int. Cl.: A61F 9/007

(54) **EYELID OPENING DEVICE**

(30) Priority: 15.10.2020 JP 2020174001
(71) Applicant: Mirai Eye Inc., Hyogo 663-8113 (JP)
(72) Inventor: AKURA Junsuke, Nishinomiya-shi, Hyogo 663-8113 (JP); TSUDA Motohumi, Kawasaki-shi, Kanagawa 216-0033 (JP)
(74) Representative: Viering, Jentschura & Partner mbB Patent- und Rechtsanwälte
(86) International application number: PCT/JP2021/037613
(87) International publication number: WO 2022/080325

(57) **Abstract**

An object of the present invention is to provide an eyelid opening device capable of easily and safely opening eyelids. When installing the eyelid opening device on eyelids G, by closing the gripping portion 1, the opening direction of the first hooking portion 31 and that of the second hooking portion 32 are changed from the upper side or the lower side to the rear while decreasing the distance between the first hooking portion 31 and the second hooking portion 32 by the arm portion 22 of the first arm portion 21 and the second arm portion 22. Then, the gripping portion is opened to change the opening direction from the rear side to the upper side or the lower side by gradually increasing the distance between the first hooking portion 31 and the second hooking portion 32 via the first arm portion 21 and the second arm portion 22. Thus, the eyelid can be opened easily and safely while the first hooking portion 31 and the second hooking portion 32 are simultaneously and smoothly hooked on the eyelid edges of the upper eyelid G1 and the lower eyelid G2.

## Description

### Technical Field

The present invention relates to an eyelid opening device used to open eyelids in ophthalmic surgery, such as, e.g., cataract surgery.

### Background Art

Conventionally, when performing ophthalmic surgery, such as, e.g., cataract surgery, inner sides of the eyelids and the surrounding portion thereof are disinfected, a drape is placed, and thereafter, the eyelids are opened with an eyelid opening device to thereby expand the surgical field.

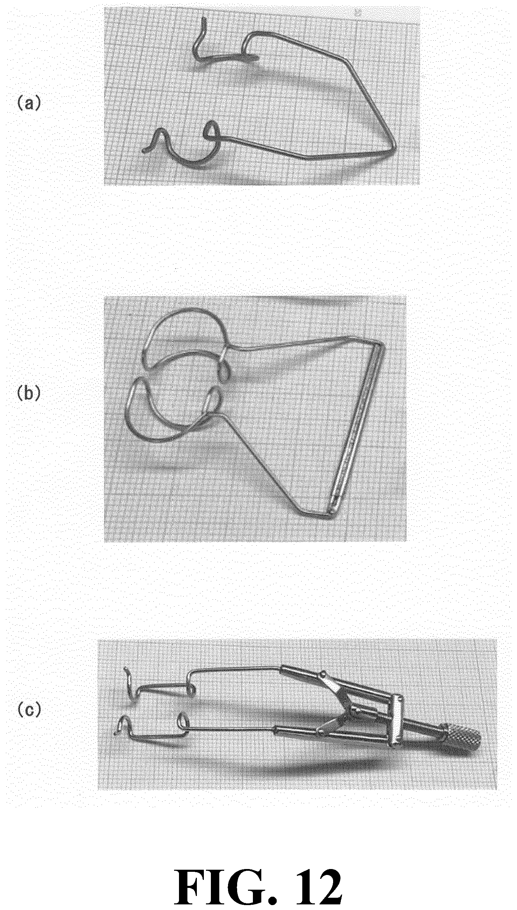

This eyelid opening device is generally composed of an arm portion provided in a state parallel to a plane direction of eyelids and a pair of hooking portions provided at the tip end portions of the arm portion. The opening directions of the hooking portions are formed to face outward in directions opposite to each other. As an eyelid opening device, as shown in FIG. 12, depending on a method of opening and closing hooking portions, various types, such as, e.g., a spring type, a screw type, and a slide type, are known.

Among these, in a spring-type eyelid opening device, the base end portion of the arm portion is bent in a U-shape or a V-shape. By opening and closing the arm portion while utilizing the elastic force of the spring in the bent portion, the distance between the hooking portions provided at the tip end portions of the arm portion is increased or decreased. Specifically, when the arm portion is closed by gripping the arm portion against the elastic force of the spring of the bent portion, the distance between the pair of hooking portions provided at the tip end portions of the arm portion gradually decreases, and the pair of hooking portions are hooked on the eyelids as it is. Thereafter, when opening the arm portions by the elastic spring force of the bent portion by weakening the gripping force of the arm portion, the distance between the hooking portions gradually increases, which opens the eyelids in the up-down direction (see, for example, Patent Document 1).

Further, in a screw-type eyelid opening device, a screw mechanism is provided at the base end portion of the arm portion. By opening and closing the arm portion with the screw mechanism, the distance between the hooking portions is decreased or increased.

Further, a slide-type opening and closing device is provided with a slide mechanism at the base end portion of the arm portion. By opening and closing the arm portion with the slide mechanism, the hooking portions are narrowed or widened.

### Prior Art Document

### Patent Document

Patent Document 1: Japanese Unexamined Patent Application Publication No. 2010-227457

### SUMMARY OF THE INVENTION

### Problems to be Solved by the Invention

However, in a conventional eyelid opening device, the arm portion opens and closes along the plane direction of the upper eyelid and the lower eyelid. Therefore, the distance between the hooking portions is decreased or increased along the plane direction of the eyelids in accordance with the opening and closing of the arm portion, and therefore, the opening directions of the hooking portions remain oriented in the plane direction of the eyelids. For this reason, even if a pair of hooking portions are to be hooked on the upper eyelid and the lower eyelid, the openings of the hooking portions remain oriented in the plane direction of the eyelids, whereas the rear sides of the actual eyelids are inclined obliquely rearward along the eyeball wall. Therefore, it is difficult to simultaneously hook the hooking portions on the upper eyelid and the lower eyelid. Moreover, the hooking portion has a constant length from the tip end portion to the base end portion on the arm portion side, while the contours of the actual upper and lower eyelids are curved in a gentle V-shape. This also makes it difficult to simultaneously hook the hooking portion on the upper eyelid and the lower eyelid. For this reason, at the time of the actual operation, in order to hook the hooking portions on the eyelids, it was required to incline the entire eyelid opening device in a state in which the distance between the hooking portions is kept decreased and hook one of the hooking portions closer to the eyelid on the upper eyelid or the lower eyelid, and then hook the other hooking portion on the other of the upper eyelid or the lower eyelid. Moreover, when the arm portions are closed to decrease the distance between the hooking portions, it is not easy to narrow the end portions (arm portion sides) of the hooking portions. As a consequence, the hooking portions are hooked on the eyelids by inclining the hooking portions in various directions or opening the eyelids with a hand opposite to a hand holding the eyelid opening device, which is troublesome to install the eyelid opening device.

Further, in the conventional eyelid opening device (particularly a spring-type eyelid opening device), when the distance between the pair of hooking portions is decreased, the ends of hooking portions on the ear side (arm portion side) are less likely to be narrowed. On the other hand, when the distance between the hooking portions is increased, the end portions of the hooking portions on the nasal side are less likely to be opened.

Further, in a patient with deep eyes (a patient with thick subcutaneous tissue or a patent with deep eyes, and the eyeball is positioned at a relatively deep position with respect to the eyelid surface), the conjunctival sac under the eyelid is located deeper along the eyeball wall toward the obliquely rear deep side. For this reason, when it is tried to open the eyelids by the conventional eyelid opening device (an eyelid opening device in which the distance between the hooking portions is decreased or increased with the opening directions of the hooking portions faced in the plane direction of the eyelids), the hooking portions lift up the eyelids forward excessively. As a result, the depth of the eye is increased, making it difficult to perform the operation, or the visibility is deteriorated by increasing the water accumulation, or the eyelid tissues are damaged to induce postoperative eyelid drooping.

An object of the present invention is to provide an eyelid opening device capable of easily and safely opening eyelids of various patients in ophthalmic surgery, such as, e.g., cataract surgery.

### Means for Solving the Problems

According to the present invention, in order to attain the above-described object, an eyelid opening device used to open eyelids in ophthalmic surgery includes:
a first hooking portion configured to hook on an eyelid edge of an upper eyelid;
a first hooking portion configured to hook on an eyelid edge of an upper eyelid;
a second hooking portion configured to hook on an eyelid edge of a lower eyelid; and
a gripping mechanism configured to decrease and increase a distance between the first hooking portion and the second hooking portion,
wherein when decreasing the distance between the first hooking portion and the second hooking portion, the gripping mechanism changes an opening direction of the first hooking portion from an upper side of the upper eyelid to a rear side of an eyeball, and changes an opening direction of the second hooking portion from a lower side of the lower eyelid to the rear side of the eyeball, and
wherein when increasing the distance between the first hooking portion and the second hooking portion, the gripping mechanism changes the opening direction of the first hooking portion from the rear side of the eyeball to the upper side of the upper eyelid, and changes the opening direction of the second hooking portion from the rear side of the eyeball to the lower side of the lower eyelid.

According to this configuration, when installing the eyelid opening device on eyelids, in the process of changing the opening direction of the first hooking portion and the opening direction of the second hooking portion from an upper side or the lower side to the rear side while decreasing the distance between the first hooking portion and the second hooking portion by the gripping mechanism, the first hooking portion and the second hooking portion are inserted into the conjunctival sac of the upper eyelid and the conjunctival sac of the lower eyelid by simultaneously sliding them to open the eyelids in a state in which the first hooking portion and the second hooking portion are hooked on the upper eyelid and the lower eyelid, respectively. Thus, it is possible to easily and safely open the eyelids of various kinds of patients.

Further, it may be configured such that the gripping mechanism is provided with
a gripping portion extending in an up-down direction on an ear side or a nasal side of the eyelids than the eyelids,
a first arm portion extending from an upper side portion of the gripping portion toward an upper eyelid side, the first arm portion being connected to the first hooking portion, and
a second arm portion extending from a lower side portion of the gripping portion toward a lower eyelid side, the second arm portion being connected to the second hooking portion,
wherein when the upper side portion and the lower side portion of the gripping portion are closed in an approaching direction, the first arm portion and the second arm portion are axially rotated relative to the eyelids while approaching each other, in accordance with it, while decreasing the distance between the first hooking portion and the second hooking portion, the opening direction of the first hooking portion is changed from the upper side of the upper eyelid to the rear side of the eyeball, and the opening direction of the second hooking portion is changed from the lower side of the lower eyelid to the rear side of the eyeball, and
wherein when the upper side portion and the lower side portion of the gripping portion are opened in a separating direction, the first arm portion and the second arm portion are axially rotated relative to the eyelids while separating from each other, in accordance with it, while increasing the distance between the first hooking portion and the second hooking portion, the opening direction of the first hooking portion is changed from the rear side of the eyeball to the upper side of the upper eyelid, and the opening direction of the second hooking portion is changed from the rear side of the eyeball to the lower side of the lower eyelid.

According to this configuration, when installing the eyelid opening device on eyelids, in the process of changing the opening direction of the first hooking portion and the opening direction of the second hooking portion from an upper side or the lower side to the rear side while decreasing the distance between the first hooking portion and the second hooking portion via the first arm portion and the second arm portion by closing the gripping portion, and then changing the opening direction from the rear side to the upper side or the lower side while gradually increasing the distance between the first hooking portion and the second hooking portion via the first arm portion and the second arm portion by opening the gripping portion, the first hooking portion and the second hooking portion can be easily inserted into the conjunctival sacs of the upper eyelid and the lower eyelid by simultaneously sliding them, and it is possible to easily open eyelids in a state in which the eyelid opening device is hooked on the upper eyelid and the lower eyelid.

Further, it may be configured such that the gripping portion is formed in an inverted V-shape along an up-down direction.

According to this configuration, since the upper side portion and the lower side portion of the gripping portion can be opened and closed in the approaching-separating direction in a simple and reliable manner, the distance between the first hooking portion and the second hooking portion and the opening directions thereof can be changed in a simple and reliable manner.

Further, it may be configured such that the gripping portion is formed of a member having an elastic force to open the upper side portion and the lower side portion. According to this configuration, when the gripping force is weakened from the state in which the gripping portion is closed, the gripping portion automatically opens in the separating direction by the elastic force of the gripping portion, and therefore, it is possible to smoothly open the eyelids.

Further, it may be configured such that the gripping portion is provided with a spring member for biasing the upper side portion and the lower side portion in the opening direction.

According to this configuration, when the gripping force is weakened from the state in which the gripping portion is closed, the gripping portion is automatically opened in the separating direction by the biasing force of the spring member of the gripping portion, and therefore, it is possible to smoothly open the eyelids.

Further, it may be configured such that the gripping portion is formed of an elastically deformable wire-like member, and the upper side portion and the lower side portion are formed in a loop shape. That is, it may be configured such that the gripping portion is formed of an elastically deformable wire-like member and is provided with
an upper side portion having one end portion connected to the first arm portion and extending toward an ear side or a nasal side than the eyelids,
a lower side portion having one end portion connected to the second arm portion and extending toward the ear side or the nasal aide than the eyelids, and
a loop portion having one end portion connected to the other end portion of the upper side portion in a folded-back manner and the other end portion connected to the other end portion of the lower side portion in a folded-back manner, the loop portion being formed in a loop shape between the upper side portion and the lower side portion, and
the upper side portion and the lower side portion are biased in the opening direction by an elastic force of the loop portion.

According to this configuration, since the upper side portion and the lower side portion of the gripping portion can be opened and closed in the approaching-separating direction in a simple and reliable manner, the distance between the first hooking portion and the second hooking portion and the opening directions thereof can be changed in a simple and reliable manner. Further, when the gripping force is weakened from the state in which the gripping portion is closed, the upper side portion and the lower side portion of the gripping portion are automatically opened by the elastic force of the loop portion, and therefore, the eyelids can be opened smoothly. Further, since the elastically deformable wire-like member is simply formed in a loop-like shape, the production cost of the eyelid opening device can be reduced.

Further, it may be configured such that the gripping portion is provided with a stopper that restricts the upper side portion and the lower side portion from opening beyond a predetermined angle.

According to this configuration, it is possible to restrict the gripping portion from opening beyond a predetermined angle.

Further, it may be configured such that the first arm portion and the second arm portion extend from the upper side portion and the lower side portion of the gripping portion to separate from each other as the first arm portion and the second arm portion advance toward the first hooking portion and the second hooking portion, respectively.

According to this configuration, when the eyelid opening device is installed on the eyelids, the gripping portion can be arranged on the rear side of the hooking portions, and therefore, the instrument manipulation during the operation is facilitated.

It may be configured such that the first hooking portion and the second hooking portion are positioned on a rear side of the first arm portion and the second arm portion.

According to this configuration, the first hooking portion and the second hooking portion can be easily inserted into the conjunctival sac of the upper eyelid and the conjunctival sac of the lower eyelid while being simultaneously slid, and the eyelids can be easily opened in a state in which the eyelid opening device is hooked on the eyelid edges of the upper eyelid and the lower eyelid. Particularly, it becomes possible to install the eyelid opening device on a patient with deep eyes.

Further, it may be configured such that the first hooking portion and the second hooking portion are each provided with a sliding portion to be inserted into a conjunctival sac on a rear side of an eyelid and an engaging portion to be hooked on an eyelid edge.

According to this configuration, it is possible to easily and safely open the eyelids with a simple structure by inserting the first hooking portion and the second hooking portion into the conjunctival sacs of the upper eyelid and the lower eyelid while sliding and hooking the engaging portion of the first hooking portion and the engaging portion of the second hooking portion on the eyelid edge of the lower eyelid and the eyelid edge of the upper eyelid.

Further, it may be configured such that the first hooking portion and the second hooking portion each maintain an inclined state within a range of an inclination angle of 25° to 45° inclined rearward with respect to a plane direction of the eyelids when the opening direction of the first hooking portion is changed from a rear side to an upper side, and the opening direction of the second hooking portion is changed from the rear side to the lower side.

According to this configuration, when the eyelids are opened by the eyelid opening device, it is possible to prevent the eyelid edge of the upper eyelid and the eyelid edge of the lower eyelid from being excessively lifted up forwardly.

Further, it may be configured such that the first hooking portion and the second hooking portion are provided with a second gripping mechanism for assisting in decreasing or increasing the distance between the first hooking portion and the second hooking portion.

According to this configuration, by opening and closing the second gripping mechanism, it is possible to supplementarily decrease or increase the distance between the first hooking portion and the second hooking portion.

Further, an eyelid opening device according to the present invention is an eyelid opening device used to open an eyelid in ophthalmic surgery, comprising:
a first hooking portion configured to hook on an eyelid edge of an upper eyelid;
a second hooking portion configured to hook on an eyelid edge of a lower eyelid; and
a coupling mechanism configured to decrease and increase a distance between the first hooking portion and the second hooking portion,
wherein when decreasing the distance between the first hooking portion and the second hooking portion, the coupling mechanism changes an opening direction of the first hooking portion from an upper side of an upper eyelid to a rear side of an eyeball and changes an opening direction of the second hooking portion from a lower side of a lower eyelid to the rear side of the eyeball, and
wherein when increasing the distance between the first hooking portion and the second hooking portion, the coupling mechanism changes the opening direction of the first hooking portion from the rear side of the eyeball to the upper side of the upper eyelid, and changes the opening direction of the second hooking portion from the rear side of the eyeball to the lower side of the lower eyelid.

According to this configuration, when installing the eyelid opening device on the eyelids, in the process of changing the opening direction of the first hooking portion and the opening direction of the second hooking portion from the upper side or the lower side to the rear side by decreasing the distance between the first hooking portion and the second hooking portion by the coupling mechanism, and then changing the opening direction from the rear side to the upper side to the lower side by gradually increasing the distance between the first hooking portion and the second hooking portion by the coupling mechanism, by simultaneously inserting the first hooking portion and the second hooking portion into the conjunctival sac of the upper eyelid and the conjunctival sac of the lower eyelid by sliding and by opening the eyelid edge of the upper eyelid and the eyelid edge of the lower eyelid in a state in which the first hooking portion and the second hooking portion are hooked on the eyelid edge of the upper eyelid and the eyelid edge of the lower eyelid, eyelids of any patients can be opened easily and safely.

### Effects of the Invention

According to the present invention, when installing the eyelid opening device on the eyelids, the opening direction of the first hooking portion and the opening direction of the second hooking portion are changed by the gripping mechanism from the upper side or the lower side to the rear side by decreasing the distance between the first hooking portion and the second hooking portion by the griping mechanism, and then the opening direction of the first hooking portion and the opening direction of the second hooking portion are changed from the rear side to the upper side or the lower side by gradually increasing the distance between the first hooking portion and the second hooking portion by the gripping mechanism. Thus, the first hooking portion and the second hooking portion are inserted into the conjunctival sac of the upper eyelid and the conjunctival sac of the lower eyelid to open the eyelids in a state in which the eyelid opening device is hooked on the eyelid edge of the upper eyelid and the eyelid edge of the lower eyelid. With this, it is possible to easily and safely open the eyelids of any patients.

Further, when the distance between thee hooking portions is increased, both the tip end portion and the base end portion of the hooking portion are configured to be easily opened, so that an adequate field of view can be ensured during the operation.

Further, since it is not required to lift the eyelids by the hooking portions forward or a load of such an operation is reduced, when installing the eyelid opening device on a person with deep eyes, it is possible to prevent the operation from making it difficult by enhancing the deep eyes, the water accumulation from increasing to reduce the visibility, or the eyelid tissue from being damaged to induce the postoperative eyelid drooping.

### BRIEF DESCRIPTION OF THE DRAWINGS

FIG. 1 is a perspective view of an eyelid opening device according to one embodiment of the present invention.
FIG. 2 is (a) a plan view, (b) a side view, and (c) a bottom view of the eyelid opening device shown in FIG. 1.
FIG. 3 is (a) a rear view and (b) a front view, and (c) an explanatory angle diagram of the eyelid opening device shown in FIG. 1.
FIG. 4 is (a) a plan view, (b) a rear view, (c) a front view of the eyelid opening device for explaining the operation of the eyelid opening device shown in FIG. 1.
FIG. 5 is a plan view showing a process of opening eyelids by the eyelid opening device shown in FIG. 1.
FIG. 6 is a front view showing a process of opening eyelids by the eyelid opening device shown in FIG. 1.
FIG. 7 is a plan view of the eyelid opening device according to another embodiment.
FIG. 8 is a perspective view of the eyelid opening device according to another embodiment.
FIG. 9 is a perspective view of the eyelid opening device according to another embodiment.
FIG. 10 is a perspective view of an eyelid opening device according to a second embodiment.
FIG. 11 is a plan view of the eyelid opening device shown in FIG. 10.
FIG. 12 is a photograph showing conventional eyelid opening devices.

### EMBODIMENTS FOR CARRYING OUT THE INVENTION

### <First Embodiment>

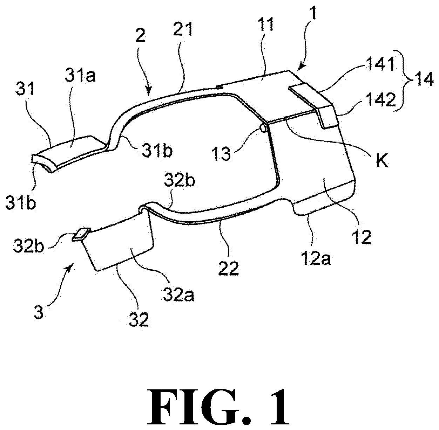

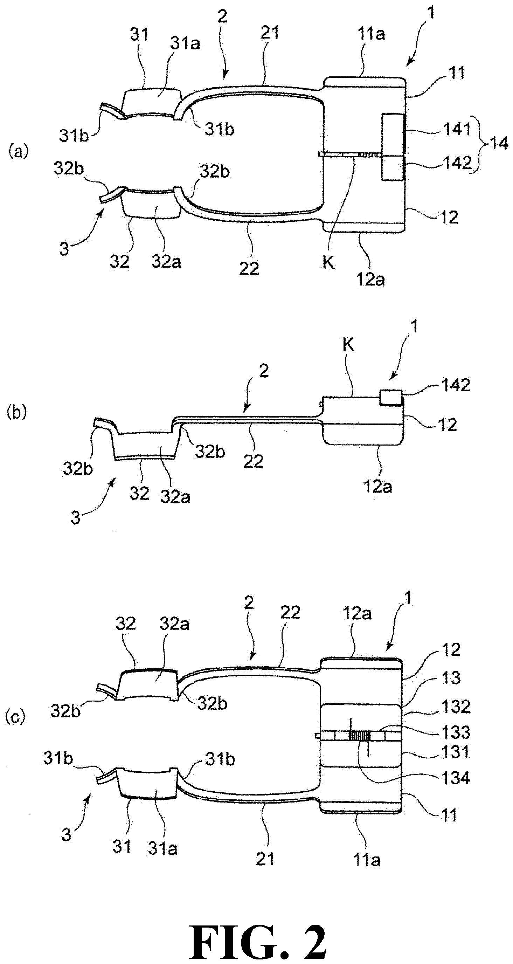

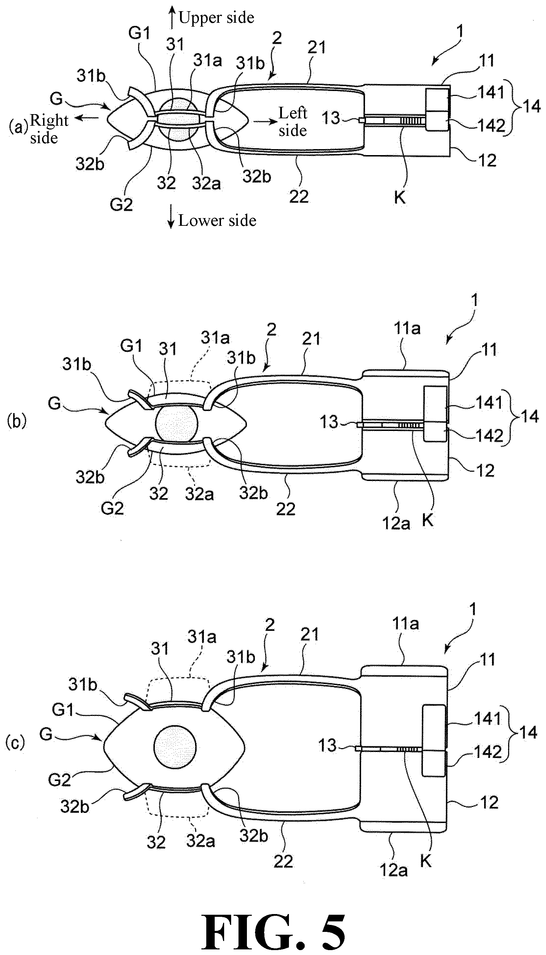

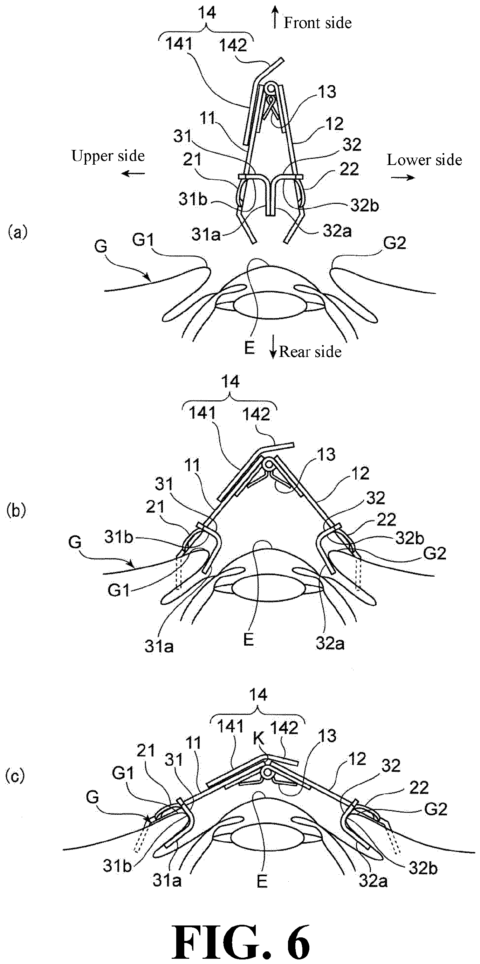

Next, a first embodiment of an eyelid opening device according to the present invention will be described with reference to FIG. 1 to FIG. 6. Note that in the present invention, the eyelids G of the patient undergoing surgery include an upper eyelid G1 and a lower eyelid G2. The direction along which the upper eyelid G1 and the lower eyelid G2 are aligned is denoted as an up-down direction (the up-down direction in FIG. 5(a), the left-right direction in FIG. 6(a)). The direction along which the eyelids G extend is denoted as a left-right direction (the left-right direction in FIG. 5 (a)), and the direction perpendicular to the eyelids G is denoted as a front-rear direction (the up-down direction of FIG. 6 (a)).

### [Configuration of Eyelid Opening Device]

As shown in FIG. 1, this eyelid opening device is provided with a gripping portion 1, an arm portion 2 provided to the gripping portion 1, and a hooking portion 3 provided at the tip end portion of the arm portion 2.

The gripping portion 1 is formed in an inverted V-shape bent in the up-down direction as a whole. The gripping portion 1 is provided with a first gripping plate 11 (upper side portion) to be arranged on an upper side, a second gripping plate 12 (lower side portion) to be arranged on a lower side, and a hinge portion 13 connecting the first gripping plate 11 and the second gripping plate 12 in an openable and closable manner.

The first gripping plate 11 and the second gripping plate 12 each are a plate-like member formed in a rectangular shape in plan view and are abutted against each other while forming an inverted V-shape. Note that the portion where the first gripping plate 11 and the second gripping plate 12 are abutted against each other forms an apex K of the inverted V-shape of the gripping portion 1.

Further, in the first gripping plate 11, the upper end portion 11a is bent rearward on the face side of the patient, while in the second gripping plate 12, the lower end portion 12a is bent rearward on the face side of the patient. Therefore, by gripping the upper end portion 11a of the first gripping plate 11 and the lower end portion 12a of the second gripping plate 12 with fingers, the gripping portion 1 is easily opened and closed with the apex K of the inverted V-shape as a pivot.

The hinge portion 13 is provided with a first hinge piece 131 provided on the rear surface of the first gripping plate 11, a second hinge piece 132 provided on the rear surface of the second gripping plate 12, and a shaft 133 supporting the first hinge piece 131 and the second hinge piece 132 in a pivotable manner. The first gripping plate 11 and the second gripping plate 12 are configured to open and close via the hinge portion 13 in accordance with the opening and closing operation of the first hinge piece 131 and the second hinge piece 132 about the shaft 133.

Further, the hinge portion 13 is provided with a spring member 134 on the rear surface thereof, and the spring member 134 urges the first gripping plate 11 and the second gripping plate 12 to open about the apex K of the gripping portion 1 as a pivot.

Note that in the gripping portion 1, a stopper 14 is provided on the front surface side of the apex K. This stopper 14 is generally formed in an inverted V-shape along the up-down direction as a whole and is composed of a first stopper piece 141 fixed to the surface of the first gripping plate 11 and a second stopper piece 142 that comes into contact with the surface of the second gripping plate 12. With this configuration, when the first gripping plate 11 and the second gripping plate 12 are opened by the biasing force of the hinge portion 13 in the separating direction, the second stopper piece 142 of the stopper 14 comes into contact with the second gripping plate 12. This makes it possible to restrict the first gripping plate 11 and the second gripping plate 12 from opening beyond a predetermined angle (the bent angle of the stopper 14).

The arm portion 2 is provided with an elongated plate-like first arm portion 21 provided to the first gripping plate 11 of the gripping portion 1, and an elongated plate-like second arm portion 22 provided to the second gripping plate 12 of the gripping portion 1.

The first arm portion 21 extends from the first gripping plate 11 of the gripping portion 1 along the left-right direction on the side of the upper eyelid G1. Further, the second arm portion 22 extends from the second gripping plate 12 of the gripping portion 1 along the left-right direction on the side of the lower eyelid G2. Thus, the first arm portion 21 and the second arm portion 22 extend substantially parallel to the left-right direction of the eyelids G with a predetermined distance from each other.

The hooking portion 3 is provided with a first hooking portion 31 provided at the tip end portion of the first arm portion 21 and a second hooking portion 32 provided at the tip end portion of the second arm portion 22.

The first hooking portion 31 is provided with a plate-shaped sliding portion 31a to be inserted into a conjunctival sac on the rear side of the upper eyelid G1, and an engaging portion 31b to be hooked on the eyelid edge of the upper eyelid G1. The sliding portion 31a extends while inclining obliquely rearward and upward with respect to the upper eyelid G1. Further, the engaging portion 31b extends from the tip end portion and the base end portion of the sliding portion 31a while being bent obliquely forward and upward.

The second hooking portion 32 is provided with a plate-shaped sliding portion 32a to be inserted into a conjunctival sac on the rear side of the lower eyelid G2 and an engaging portion 32b to be hooked on the eyelid edge of the lower eyelid G2. The sliding portion 32a extends while inclining obliquely rearward and downward with respect to the lower eyelid G2. Further, the engaging portion 32b extends from the tip end portion and the base end portion of the sliding portion 32a while being bent obliquely forward and downward.

Thus, when the first hooking portion 31 and the second hooking portion 32 are hooked on the eyelid edge of the upper eyelid G1 and the eyelid edge of the lower eyelid G2, respectively, the sliding portion 31a is inserted into the conjunctival sac of the upper eyelid G1 obliquely rearward and upward, and the sliding portion 32a is inserted into the conjunctival sac of the lower eyelid G2 obliquely rearward and downward. Further, the engaging portion 31b and the engaging portion 32b are hooked on the eyelid edge of the upper eyelid G1 and the eyelid edge of the lower eyelid G2, respectively. Thus, the eyelid edge of the upper eyelid G1 and the eyelid edge of the lower eyelid G2 are placed between the sliding portion 31a and and the engaging portion 31b and between the sliding portion 32a and the engaging portion 32b, respectively.

Further, the first hooking portion 31 and the second hooking portion 32 are connected to the first arm portion 21 and the second arm portion 22 via the engaging portion 31b and the engaging portion 32b on the base end portion side, respectively. Therefore, when the eyelid opening device is installed on the eyelids G, the first hooking portion 31 and the second hooking portion 32 are positioned on the rear side of the first arm portion 21 and the second arm portion 22. Therefore, the first hooking portion 31 and the second hooking portion 32 can be easily and simultaneously inserted into the conjunctival sac of the upper eyelid G1 and the conjunctival sac of the lower eyelid G2 while being slid. Further, the eyelids G can be easily opened in a state in which the eyelid opening device is hooked on the eyelid edges of the upper eyelid G1 and the lower eyelid G2. Particularly, the eyelid opening device can be easily installed on a patient with deep eyes.

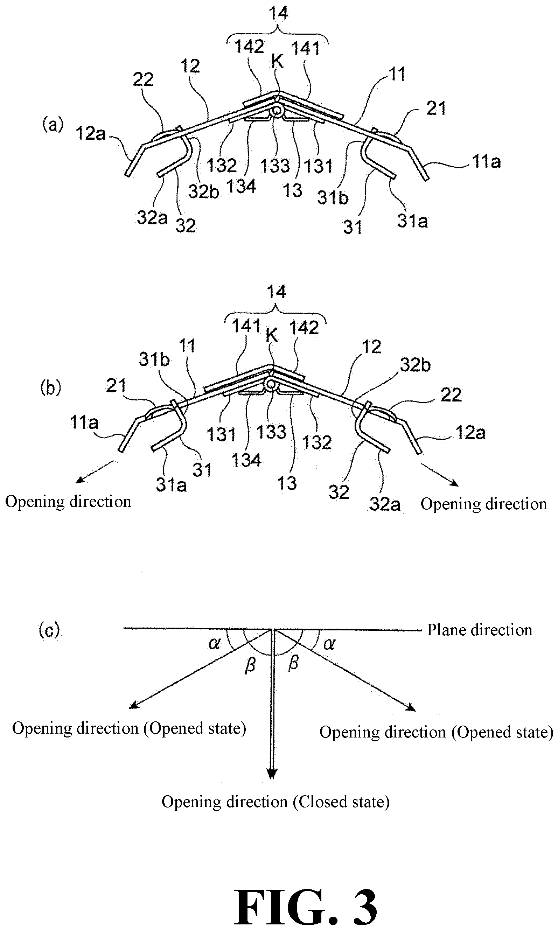

Note that, as shown in FIG. 3(b), the first hooking portion 31 and the second hooking portion 32 are formed in a U-shape in a front view by the sliding portion 31a, 32a and the engaging portion 31b, 32b. The direction in which the U-shape is opened (in this embodiment, the upwardly or downwardly extending direction of the sliding portion 32a) is defined as an opening direction.

### [Operation of Eyelid Opening Device]

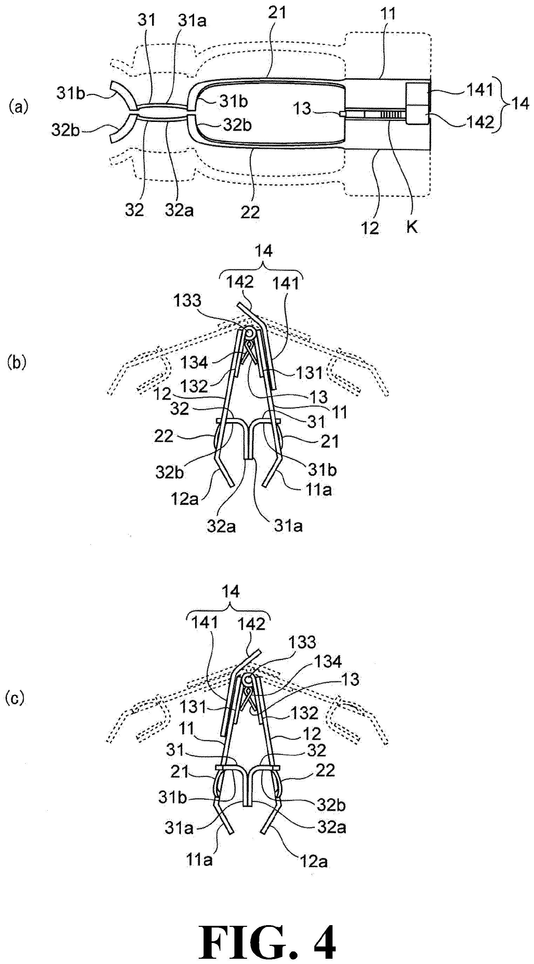

Next, the operation of this eyelid opening device will be described with reference to FIG. 4.

First, in the natural state in which the eyelid opening device is opened (see the dotted line in FIG. 4), the first gripping plate 11 and the second gripping plate 12 of the gripping portion 1 are gripped, and a gripping force is applied in the inward direction against the biasing force of the spring member 134 of the hinge portion 13. With this, as shown by the solid line in FIG. 4, the first gripping plate 11 and the second gripping plate 12 are closed in the approaching direction with the inverted V-shaped apex K as a pivot so that the gripping portion 1 is bent rearward.

When the first gripping plate 11 and the second gripping plate 12 are moved in the approaching direction as described above, the first arm portion 21 and the second arm portion 22 rotate within a range of about 90 degrees relative to the eyelids G while approaching each other. At this time, as shown by the solid line in FIG. 4 (c), the first hooking portion 31 and the second hooking portion 32 are changed from the laying state to the upright state with respect to the eyelids G while decreasing the distance between the first hooking portion 31 and the second hooking portion 32. In accordance with the change of the state, the opening direction of the first hooking portion 31 and the opening direction of the second hooking portion 32 are each changed from the upper side or the lower side to the rear side. Finally, the eyelid opening device is shifted to the closed state (the solid line in FIG. 4). At this time, the opening direction of the first hooking portion 31 and the opening direction of the second hooking portion 32 are oriented in the same rearward direction.

On the other hand, in a state in which the eyelid opening device is closed (the solid line in FIG. 4), when the gripping force with respect to the gripping portion 1 is weakened, as shown by the dotted line in FIG. 4, the first gripping plate 11 and the second gripping plate 12 are opened in the separating direction with the inverted V-shaped apex K as a pivot such that the gripping portion 1 returns to the original opened state by the biasing force of the spring member 134 of the hinge portion 13.

When the first gripping plate 11 and the second gripping plate 12 are opened in the separating direction, the first arm portion 21 and the second arm portion 22 are axially rotated within a range of 90 degrees relative to the eyelids G while separating from each other. At this time, as shown by the dotted line in FIG. 4 (c), the first hooking portion 31 and the second hooking portion 32 change their postures from the upright state to the laying state with respect to the eyelids G while increasing the distance between the first hooking portion 31 and the second hooking portion 32. In accordance with the change of the posture, the opening direction of the first hooking portion 31 and the opening direction of the second hooking portion 32 are each changed from the rear side to the upper side or the lower side and finally shifted to the state in which the eyelid opening device is in an opened state (the dotted line in FIG. 4). At this time, the opening direction of the first hooking portion 31 and the opening direction of the second hooking portion 32 are directed to a direction opposite to the upward direction and a direction opposite to the downward direction, respectively.

Note that in this embodiment, in the natural state (the dotted line in FIG. 4) in which the eyelid opening device is fully opened, the first gripping plate 11 and the second gripping plate 12 of the gripping portion 1 are inclined rearward with respect to the plane direction of the eyelids (the plane direction including the up-down direction in FIG. 5 and the left-right direction in FIG. 5). In accordance with it, the opening direction of the first hooking portion 31 and the opening direction of the second hooking portion 32 are also inclined in an upward direction and a downward direction in a rearwardly inclined state with respect to the plane direction of the eyelids G, respectively. The inclination angle α of the opening direction of the first hooking portion 31 and the inclination angle α of the opening direction of the second hooking portion 32 each are not particularly limited, but preferably within an inclination angle of 25° to 45° with respect to the plane direction of the eyelids G (see FIG. 3 (c)).

Further, in the completely closed state (the solid line in FIG. 4) of the eyelid opening device, the first gripping plate 11 and the second gripping plate 12 of the gripping portion 1 each are inclined in a closely vertical state with respect to the plane direction of the eyelids G. In accordance with it, the opening direction of the first hooking portion 31 and the opening direction of the second hooking portion 32 each are inclined in a state close to or substantially perpendicular to the plane direction of the eyelids G. The inclination angle β of the opening direction of the first hooking portion 31 and the inclination angle β of the second hooking portion 32 at this time each are not particularly limited, but preferably within an inclination angle of 60° to 90° with respect to the plane direction of the eyelids G (see FIG. 3 (c)).

### [Installation Method of Eyelid Opening Device]

Next, the method of installing the eyelid opening device on eyelids G will be described.

First, as shown in FIG. 5 (a) and FIG. 6 (a), in a state in which the eyelid opening device is placed at a predetermined distance in front of the eyelids G, when the first gripping plate 11 and the second gripping plate 12 in a fully opened natural state of the gripping portion 1 are closed in the approaching direction against the biasing force of the spring member 134 with the inverted V-shaped apex K as a pivot, the first arm portion 21 and the second arm portion 22 are relatively rotated with respect to the eyelids G while approaching each other. In accordance with it, the first hooking portion 31 and the second hooking portion 32 change in posture from the laying state to the upright state with respect to the eyelids G while decreasing the distance between the first hooking portion 31 and the second hooking portion 32. Thus, the eyelid opening device is shifted to the fully closed state. At this time, the opening direction of the first hooking portion 31 and the opening direction of the second hooking portion 32 are changed from the upper side to the rear side and from the lower side to the rear side with respect to the eyelids G, respectively.

Then, as shown in FIG. 5 (b) and FIG. 6 (b), at the time of engaging the eyelid opening device on the eyelids G, when the first gripping plate 11 and the second gripping plate 12 of the gripping portion 1 in closed state are gradually opened in the separating direction by the biasing force of the spring member 134 with the inverted V-shaped apex K as a pivot, the first arm portion 21 and the second arm portion 22 are rotated relative to the eyelids G while separating from each other. In accordance with it, the first hooking portion 31 and the second hooking portion 32 gradually change the posture from the upright state to the inclined state with respect to the eyelids G while increasing the distance therebetween. At this time, the opening direction of the first hooking portion 31 and the opening direction of the second hooking portion 32 each are changed from the rear side with respect to the eyelids G to the obliquely rearward and upward and the obliquely rearward a downward with respect to the eyelids G, respectively. Therefore, the sliding portion 31a of the first hooking portion 31 and the sliding portion 32a of the second hooking portion 32 are simultaneously inserted into the conjunctival sac on the rear side of the upper eyelid G1 obliquely upward and the conjunctival sac on the rear side of the lower eyelid G2 obliquely downward, respectively.

Then, as shown in FIG. 5 (c) and FIG. 6 (c), when opening the eyelids G with the eyelid opening device, the first gripping plate 11 and the second hooking portion 32 of the gripping portion 1 in the half-opened state are further opened in the separating direction by the biasing force of the spring member 134 with the inverted V-shaped apex X as a pivot. With this, the first arm portion 21 and the second arm portion 22 are relatively rotated with respect the eyelids G while separating to each other. In accordance with the movements, the first hooking portion 31 and the second hooking portion 32 are changed in posture from the inclined state to the laying state while increasing the distance therebetween. At this time, the opening direction of the first hooking portion 31 and the opening direction of the second hooking portion 32 are changed from the obliquely rearward and upward side and the obliquely rearward and downward side with respect to the eyelids G to the upward side and the downward side, respectively. Therefore, the upper eyelid G1 and the lower eyelid G2 are opened to predetermined positions while the engaging portion 3 1b of the first hooking portion 31 and the engaging portion 32b of the second hooking portion 32 are hooked on the eyelid edge of the upper eyelid G1 and the eyelid edge of the lower eyelid G2, respectively. Thereafter, the gripping portion 1 of the eyelid opening device is placed on the face on the lateral side of the eyeball E or on a drape to keep the opened state of the upper eyelid G1 and the lower eyelid G2.

As described above, when the eyelid opening device is installed on the eyelids G, in the process of changing the opening direction from the upper side or the lower side to the rear side while decreasing the distance between the first hooking portion 31 and the second hooking portion 32 via the first arm portion 21 and the second arm portion 22 by closing the gripping portion 1, and then changing the opening directions from the rear side to the upper side or the lower side while gradually increasing the distance between the first hooking portion 31 and the second hooking portion 32 via the first arm portion 21 and the second arm portion 22 by opening the gripping portion 1, the first hooking portion 31 and the second hooking portion 32 are slidably inserted into the conjunctival sac of the upper eyelid G1 and the conjunctival sac of the lower eyelid G2 at the same time to open the eyelids in a state in which the eyelid edge of the upper eyelid G1 and the eyelid edge of the lower eyelid G2 are engaged by the first hooking portion 31 and the second hooking portion 32. With this, the eyelids G can be opened easily and safely.

Note that when removing the eyelid opening device after surgery, the eyelid opening device can be easily and assuredly removed from the eyelids G by performing the operation opposite to the above-described installation of the eyelid opening device.

In this embodiment, the first arm portion 21 and the second arm portion 22 of the gripping portion 1 are configured as a gripping mechanism, but the gripping mechanism may be another configuration. In short, the configuration is not limited as long as it is configured such that when decreasing the distance between the first hooking portion 31 and the second hooking portion 32, the opening direction of the first hooking portion 31 and the opening direction of the second hooking portion 32 are changed from the upper side or the lower side to the rear side with respect to the eyelids G, respectively, and when increasing the distance between the first hooking portion 31 and the second hooking portion 32, the opening direction of the first hooking portion 31 and the opening direction of the second hooking portion 32 are changed from the rear side to the upper side or the lower side with respect to the eyelids G, respectively.

Further, although the gripping portion 1 is formed in an inverted V-shape bent along the up-down direction, it may be formed in an inverted V-shape gently curved in the up-down direction. Alternatively, the gripping portion 1 may have a flat shape in an open natural state and may transit to an inverted V-shape in a closed state in which a gripping force is applied.

Further, although the gripping portion 1 is biased by the spring member 134 in a direction to open the first gripping plate 11 and the second gripping plate 12, the elastic force of the gripping portion 1 may be biased in a direction to open the first gripping plate 11 and the second gripping plate 12.

Further, although the gripping portion 1 is provided with the stopper 14 that restricts the first gripping plate 11 and the second gripping plate 12 from opening beyond a predetermined angle, the stopper 14 may not be provided.

Further, although the first hooking portion 31 and the second hooking portion 32 are configured to include the sliding portions 31a and 32a to be inserted into conjunctival sacs on the rear side of the eyelids G and the engaging portions 31b and 32b to be hooked on the eyelid edges, but another configuration may be adapted.

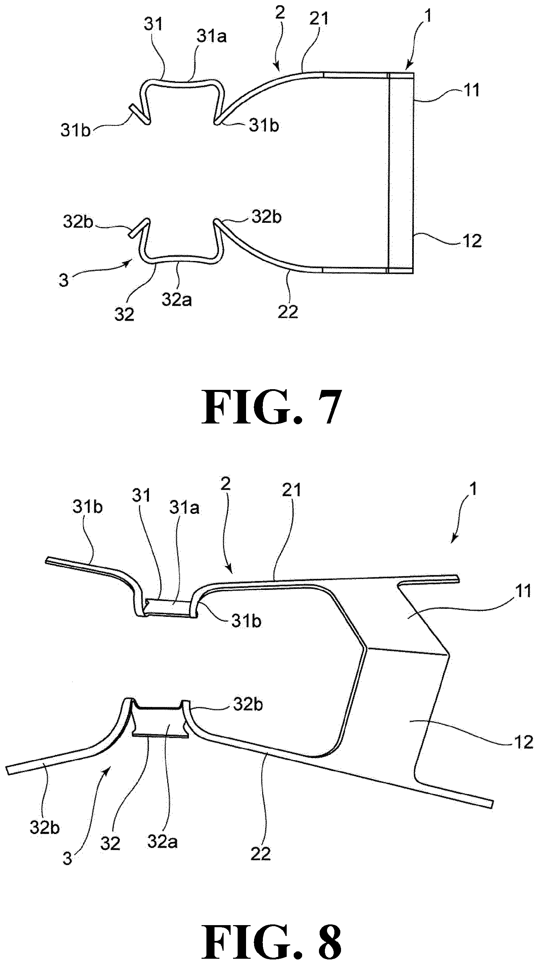

Further, although the hooking portion 3 has sliding portions 31a and 31b each formed in a plate shape, it may be formed in an elastically deformable wire-like member as shown in FIG. 7.

Further, as shown in FIG. 7 and FIG. 8, the gripping portion 1 may have a first gripping plate 11 and a second gripping plate 12 formed of a plate spring member, and the apex K at the center thereof may function as the hinge portion 13 of the gripping portion 1 shown in FIG. 1.

Further, although the first arm portion 21 and the second arm portion 22 extend parallel to each other from the first gripping plate 11 and the second gripping plate 12 of the gripping portion 1 toward the first hooking portion 31 and the second hooking portion 32, they may not necessarily extend parallel to each other. For example, as shown in FIG. 8, the first arm portion 21 and the second arm portion 22 may extend from the upper side portion and the lower side portion of the gripping portion 1 toward the first hooking portion 31 and the second hooking portion 32, respectively, so as to approach to each other. With this, when the eyelid opening device is installed on the eyelids G, the gripping portion 1 can be placed on the rear side of the first hooking portion 31 and the second hooking portion 32, thereby facilitating the instrument manipulation during the operation.

Further, the first hooking portion 31 and the second hooking portion 32 may be provided with a second gripping mechanism for assisting in decreasing or increasing the distance between the first hooking portion 31 and the second hooking portion 32. For example, as shown in FIG. 8, in the eyelid opening device, when the engaging portion 31b on the tip end side of the first hooking portion 31 and the engaging portion 32b of the second hooking portion 32 are formed to be long, the tip end portion side of the engaging portion 31b and the tip end portion side of the engaging portion 32b each function as a second gripping mechanism. With this configuration, by opening and closing the second gripping mechanism (the tip end portion side engaging portions 31b and 32b), the distance between the first hooking portion 31 and the second hooking portion 32 can be supplementarily decreased or increased.

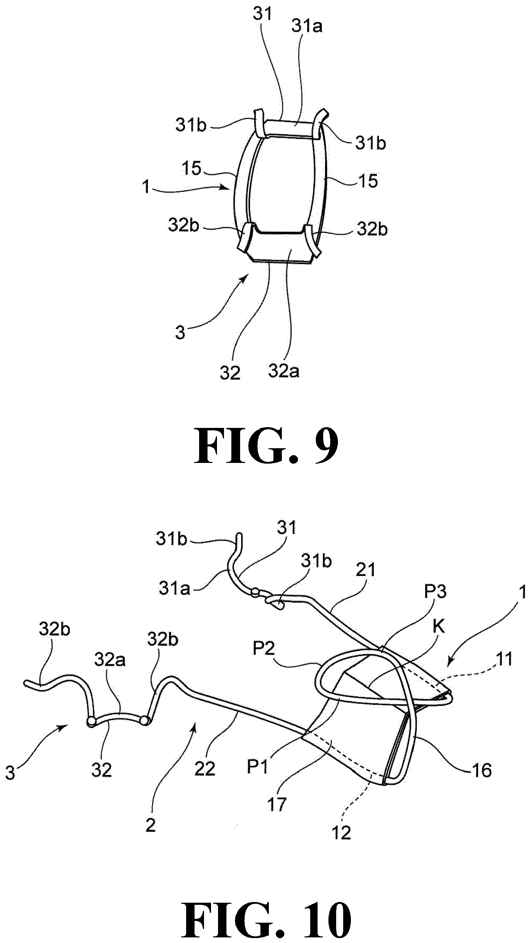

Further, in the eyelid opening device, in place of the gripping mechanism composed of the gripping portion 1 and the arm portion 2, the first hooking portion 31 and the second hooking portion 32 may be opened and closed by a coupling mechanism. Specifically, as shown in FIG. 9, this coupling mechanism is provided with two elastic connecting portions 15 and 15 having a restoring force connecting both ends of the first hooking portion 31 and the second hooking portion 32 along the up-down direction. With this configuration, when the eyelid opening device is installed on the eyelids G, by closing the connecting portions 15 and 15 of the coupling mechanism in an inverted V-shape by pressing the first hooking portion 31 and the second hooking portion 32 in the approaching direction, the opening direction of the first hooking portion 31 and the opening direction of the second hooking portion 32 are changed from the upper side or the lower side to the rearward side while decreasing the distance between the first hooking portion 31 and the second hooking portion 32. Thereafter, the connecting portions 15 and 15 of the coupling mechanism are opened by gradually weakening the pressing force to the first hooking portion 31 and the second hooking portion 32 to thereby change the opening direction from the rearward side to the upward side or the downward side by gradually increasing the distance between the first hooking portion 31 and the second hooking portion 32. Then, in the process of this operation, the sliding portion 31a of the first hooking portion 31 and the sliding portion 32a of the second hooking portion 32 are simultaneously inserted into the conjunctival sac of the upper eyelid G1 and the conjunctival sac of the lower eyelid G2 while being slid, respectively. Then, the engaging portion 31b of the first hooking portion 31 and the engaging portion 32b of the second hooking portion 32 are hooked on the eyelid of the upper eyelid G1 and the eyelid of the lower eyelid G2, respectively. With this, it is possible to easily and safely open the eyelids G in a state in which the first hooking portion 31 and the second hooking portion 32 are hooked on the eyelid edges of the eyelids G.

Note that in the eyelid opening device shown in FIG. 9, a gripping mechanism composed of the gripping portion 1 and the arm portion 2 as shown in FIG. 1 may be detachably provided. Specifically, the tip end portion of the first arm portion 21 and the tip end portion of the second arm portion 22 are detachably connected to the engaging portion 31b and the engaging portion 32b. With this configuration, after installing the eyelid opening device on the eyelids by the gripping portion 1 and the arm portion 2, it is possible to remove the gripping portion 1 and the arm portion 2 from the eyelid opening device during operation. Further, when removing the eyelid opening device from the eyelids G, the gripping portion 1 and the arm portion 2 are connected to the eyelid opening device, and then the eyelid opening device can be removed from the eyelids G by the gripping portion 1 and the arm portion 2. Further, the eyelid opening device may be installed on the eyelids G or removed from the eyelids G using equipment other than the gripping portion 1 and the arm portion 2.

The material of the eyelid opening device is not particularly limited. But, the eyelid opening device may be made of one or a plurality of materials of PMMA, urethane resin, acrylic resin, silicone rubber, polypropylene, polyester-based thermoplastic elastomer, olefin-based thermoplastic elastomer, polyacetal polymer, metal, such as, e.g., stainless steel and titanium, and resin and rubber having good shape-recovery properties.

### <Second Embodiment>

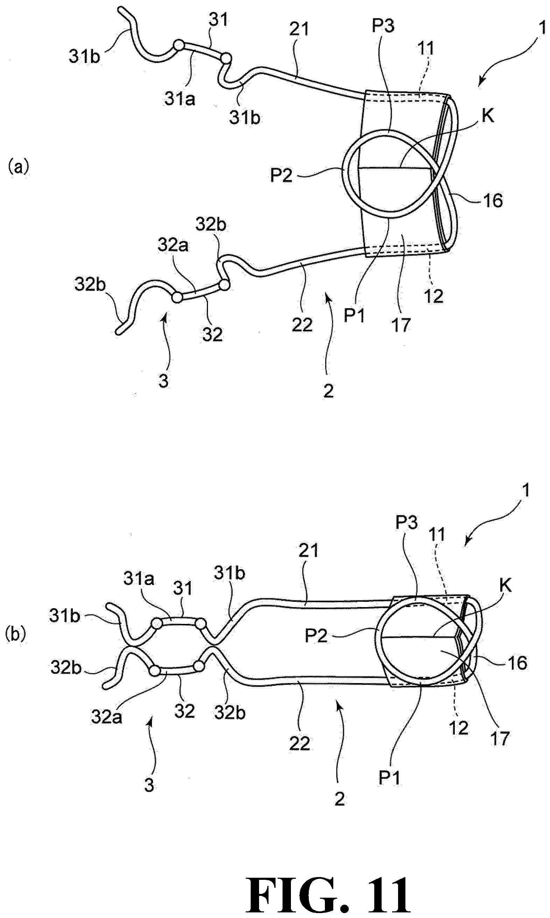

Next, a second embodiment of an eyelid opening device according to the present invention will be described with reference to FIG. 10 and FIG. 11. In the following description, only the configuration that differs from the embodiment described above will be described, and the description of the same configuration will be omitted, and the same reference symbol will be assigned.

As shown in FIG. 10, the eyelid opening device according to this embodiment is configured by bending one elastically deformable wire-like member made of metal or the like to form the gripping portion 1, the arm portion 2 (the first arm portion 21 and the second arm portion 22), and the hooking portion 3 (the first hooking portion 31 and the second hooking portion 32).

As shown in FIG. 10, the gripping portion 1 is provided with an upper side portion 11 having one end portion connected to the first arm portion 21, the upper side portion 11 extending in the left-right direction on the ear side or the nose side than the eyelids, a lower side portion 12 having the other portion connected to the second arm portion 22, the lower side portion 12 extending in the left-right direction on the ear side or the nose side than the eyelids, a loop portion 16 provided between the upper side portion 11 and the lower side portion 12, and a hinge gripping portion 17 provided on the rear side of the loop portion 16.

In the loop portion 16, one end portion is connected to the other end portion of the upper side portion 11 in a folded-back manner, the other end is connected to the other end portion of the lower side portion 12 in a folded-back manner, and a substantially circular loop-like shape is formed between the upper side portion 11 and the lower side portion 12.

The shape of the loop portion 16 will be specifically described. As shown in FIG. 11, in a plan view (left-right and up-down direction), the loop portion 16 extends from the right side other end portion of the upper side portion 11 toward the lower side portion 12 in the obliquely leftward and downward direction in FIG. 11, extends while being gently bent in the obliquely leftward and upward direction in FIG. 11 at a first bent portion P1, then extends while being gently bent in the obliquely rightward and upward direction in FIG. 11 toward the upper side portion 11 at a second bent portion P2, extends while being generally bent in the obliquely rightward and upward direction in FIG. 11 at a third bent portion P3, and is connected to the other end portion of the lower side portion 12 on the left side.

Further, in the side view (left-right and front-rear direction), the loop portion 16 extends from the other end portion of the upper side portion 11 on the left side in the obliquely leftward and frontward direction, extends while being gently bent in the obliquely leftward and rearward direction at the first bent portion P1, extends in the obliquely rightward and frontward direction while being gradually bent at the second bent portion P2, extends while being gently bent in the obliquely rightward and rearward direction at the third bent portion P3, and is connected to the other end portion of the lower side portion 12 on the left side.

Further, the loop portion 16 has a spring-like elastic force by the above-described structure, and the upper side portion 11 and the lower side portion 12 are biased to open by the elastic force of the loop portion 16. Note that in this embodiment, the loop portion 16 forms one loop, but the present invention is not limited thereto, and two or more loops may be formed. Further, the loop portion 16 may extend vertically forward from the arm portions 21 and 22.

As shown in FIG. 11 (a), in a state in which the upper side portion 11 of the gripping portion 1 and the lower side portion 12 of the gripping portion 1 are naturally opened, the first arm portion 21 and the second arm portion 22 are formed in a truncated chevron shape in which the end portions on the side of the hooking portions 3 are spaced apart than the end portions on the side of the gripping portion 1. As shown in FIG. 11 (b), when the upper side portion 11 and the lower side portion 12 of the gripping portion 1 are closed in the approaching direction, they become parallel to each other.

The hinge gripping portion 17 is a belt-like member bridged between the upper side portion 11 and the lower side portion 12. This hinge gripping portion 17 is a second gripping mechanism for assisting the gripping mechanism configured by the upper side portion 11, the lower side portion 12, and the loop portion 16. The hinge gripping portion 17 prevents the eyelid opening device from collapsing when the upper side portion 11 and the lower side portion 12 are opened and closed, and regulates that the upper side portion 11 and the lower side portion 12 are opened beyond a predetermined angle when the upper side portion 11 and the lower side portion 12 are opened. Further, since the opening mechanisms are the same and two gripping mechanisms different in the structure are provided, the spring force can be increased, and the opening and closing can be performed very smoothly.

Further, the hinge gripping portion 17 is configured to be connected by a drainage device for discharging the liquid in the eyelids G on the rear side thereof. With this configuration, the drainage device and the eyelid opening device can be integrally installed by the operation of arranging the eyelid opening device on the eyelids G.

Thus, in the natural condition in which the eyelid opening device is opened (FIG. 11 (a)), when the upper side portion 11 and the lower side portion 12 of the gripping portion 1 are gripped and a gripping force is applied in the inward direction against the biasing force of the loop portion 16, the loop portion 16 goes upward on the front side of the upper side portion 11 and the lower side portion 12, and the upper side portion 11 and the lower side portion 12 is closed in the approaching direction.

When the upper side portion 11 and the lower side portion 12 are closed in the approaching direction as described above, the first arm portion 21 and the second arm portion 22 rotate within a range of about 90 degrees relative to the eyelids G while approaching each other. At this time, the first hooking portion 31 and the second hooking portion 32 change the posture from the laying state to the upright state with respect to the eyelids G while decreasing the distance therebetween. In accordance with it, the opening direction of the first hooking portion 31 and the opening direction of the second hooking portion 32 are changed from the upper side or the lower side to the rear side, and finally, the eyelid opening device shifts to the closed state (FIG. 11 (b)). At this time, the opening direction of the first hooking portion 31 and the opening direction of the second hooking portion 32 are oriented in the same rearward direction.

On the other hand, in a state in which the eyelid opening device is closed (FIG. 11 (b)), when the gripping force with respect to the gripping portion 1 is weakened, the upper side portion 11 and the lower side portion 12 are opened in the separating direction while the loop portion 16 goes rearward to return to the original position.

Then, when the upper side portion 11 and the lower side portion 12 are opened in a separating direction as described above, the first arm portion 21 and the second arm portion 22 are axially rotated within a range of 90 degrees relative to the eyelids G while separating from each other. At this time, the first hooking portion 31 and the second hooking portion 32 change the posture from the upright state to the laying state with respect to the eyelids G while increasing the distance between the first hooking portion 31 and the second hooking portion 32. In accordance with it, the opening direction of the first hooking portion 31 and the opening direction of the second hooking portion 32 are changed from the rear side to the upper side or the lower side, and finally, the eyelid opening device is shifted to the opened state (FIG. 11 (a)). At this time, the opening direction of the first hooking portion 31 and the opening direction of the second hooking portion 32 face in opposite directions, i.e., in the upward direction and the downward direction.

According to this configuration, since the upper side portion 11 and the lower side portion 12 of the gripping portion 1 can be opened and closed in the approaching-separating direction in a simple and reliable manner, the distance between the first hooking portion 31 and the second hooking portion 32 and the opening directions thereof can be changed in a simple and reliable manner. Further, when the gripping force is reduced from the state in which the gripping portion 1 is closed, the loop portion 16 has an elastic force to automatically open the upper side portion 11 and the lower side portion 12 of the gripping portion 1 in the separating direction, and therefore, the eyelids G can be opened smoothly. Further, since the gripping portion 1 is configured such that an elastically deformable wire-like member is simply formed in a loop-like shape, the production cost of the eyelid opening device can be reduced.

The embodiments of the present invention have been described above with reference to the attached drawings, but the present invention is not limited to the illustrated embodiments. It should be understood that various modifications and variations can be made to the illustrated embodiments falling within the same scope as or equivalent to the present invention.

### Description of Symbols

- 1:: Gripping portion
11: Upper side portion (first gripping plate)
12: Lower side portion (second gripping plate)
13: Hinge portion portion
131: First hinge piece
132: Second hinge piece
133: Shaft
134: Spring member
14: Stopper
141: First stopper piece
142: Second stopper piece
15: Connecting portion
16; Loop portion
17: Hinge gripping portion
- 2:: Arm portion
21: First arm portion
22: Second arm portion
- 3:: Hooking portion
31: First hooking portion
31a: Sliding portion
31b: Engaging portion
32: Second hooking portion
32a: Sliding portion
32b: Engaging portion

## Claims

1. An eyelid opening device used to open eyelids in ophthalmic surgery, comprising:
a first hooking portion configured to hook on an eyelid edge of an upper eyelid;
a second hooking portion configured to hook on an eyelid edge of a lower eyelid; and
a gripping mechanism configured to decrease and increase a distance between the first hooking portion and the second hooking portion,
wherein when decreasing the distance between the first hooking portion and the second hooking portion, the gripping mechanism changes an opening direction of the first hooking portion from an upper side of the upper eyelid to a rear side of an eyeball, and changes an opening direction of the second hooking portion from a lower side of the lower eyelid to the rear side of the eyeball, and
wherein when increasing the distance between the first hooking portion and the second hooking portion, the gripping mechanism changes the opening direction of the first hooking portion from the rear side of the eyeball to the upper side of the upper eyelid, and changes the opening direction of the second hooking portion from the rear side of the eyeball to the lower side of the lower eyelid.

2. The eyelid opening device as recited in claim 1,
wherein the gripping mechanism is provided with
a gripping portion extending in an up-down direction on an ear side or a nasal side of the eyelids than the eyelids,
a first arm portion extending from an upper side portion of the gripping portion toward an upper eyelid side, the first arm portion being connected to the first hooking portion, and
a second arm portion extending from a lower side portion of the gripping portion toward a lower eyelid side, the second arm portion being connected to the second hooking portion,
wherein when the upper side portion and the lower side portion of the gripping portion are closed in an approaching direction, the first arm portion and the second arm portion are axially rotated relative to the eyelids while approaching each other, in accordance with it, while decreasing the distance between the first hooking portion and the second hooking portion, the opening direction of the first hooking portion is changed from the upper side of the upper eyelid to the rear side of the eyeball, and the opening direction of the second hooking portion is changed from the lower side of the lower eyelid to the rear side of the eyeball, and
wherein when the upper side portion and the lower side portion of the gripping portion are opened in a separating direction, the first arm portion and the second arm portion are axially rotated relative to the eyelids while separating from each other, in accordance with it, while increasing the distance between the first hooking portion and the second hooking portion, the opening direction of the first hooking portion is changed from the rear side of the eyeball to the upper side of the upper eyelid, and the opening direction of the second hooking portion is changed from the rear side of the eyeball to the lower side of the lower eyelid.

3. The eyelid opening device as recited in claim 2,
wherein the gripping portion is formed in an inverted V-shape along the up-down direction.

4. The eyelid opening device as recited in claim 2,
wherein the gripping portion is formed of a member having an elastic force to open the upper side portion and the lower side portion.

5. The eyelid opening device as recited in claim 2,
wherein the gripping portion is provided with a spring member for biasing the upper side portion and the lower side portion in the opening direction.

6. The eyelid opening device as recited in claim 2,
wherein the gripping portion is formed of an elastically deformable wire-like member,
wherein the gripping portion is provided with
an upper side portion having one end portion connected to the first arm portion and extending toward an ear side or a nasal side than the eyelids,
a lower side portion having one end portion connected to the second arm portion and extending toward the ear side or the nasal aide than the eyelids, and
a loop portion having one end portion connected to the other end portion of the upper side portion in a folded-back manner and the other end portion connected to the other end portion of the lower side portion in a folded-back manner, the loop portion being formed in a loop shape between the upper side portion and the lower side portion, and
wherein the upper side portion and the lower side portion are biased in the opening direction by an elastic force of the loop portion.

7. The eyelid opening device as recited in claim 2,
wherein the gripping portion is provided with a stopper that restricts the upper side portion and the lower side portion from opening beyond a predetermined angle.

8. The eyelid opening device as recited in claim 2,
wherein the first arm portion and the second arm portion extend from the upper side portion and the lower side portion of the gripping portion to separate from each other as the first arm portion and the second arm portion advance toward the first hooking portion and the second hooking portion, respectively.

9. The eyelid opening device as recited in claim 2,
wherein the first hooking portion and the second hooking portion are positioned on a rear side of the first arm portion and the second arm portion.

10. The eyelid opening device as recited in claim 1,
wherein the first hooking portion and the second hooking portion are each provided with a sliding portion to be inserted into a conjunctival sac on a rear side of an eyelid and an engaging portion to be hooked on an eyelid edge.

11. The eyelid opening device as recited in claim 1,
wherein the first hooking portion and the second hooking portion each maintain an inclined state within a range of an inclination angle of 25° to 45° inclined rearward with respect to a plane direction of the eyelids when the opening direction of the first hooking portion is changed from a rear side to an upper side, and the opening direction of the second hooking portion is changed from the rear side to the lower side.

12. The eyelid opening device as recited in claim 1,
wherein the first hooking portion and the second hooking portion are provided with a second gripping mechanism for assisting in decreasing or increasing the distance between the first hooking portion and the second hooking portion.

13. An eyelid opening device used to open eyelids in ophthalmic surgery, comprising:
a first hooking portion configured to hook on an eyelid edge of an upper eyelid;
a second hooking portion configured to hook on an eyelid edge of a lower eyelid; and
a coupling mechanism configured to decrease and increase a distance between the first hooking portion and the second hooking portion,
wherein when decreasing the distance between the first hooking portion and the second hooking portion, the coupling mechanism changes an opening direction of the first hooking portion from an upper side of an upper eyelid to a rear side of an eyeball and changes an opening direction of the second hooking portion from a lower side of a lower eyelid to the rear side of the eyeball, and
wherein when increasing the distance between the first hooking portion and the second hooking portion, the coupling mechanism changes the opening direction of the first hooking portion from the rear side of the eyeball to the upper side of the upper eyelid, and changes the opening direction of the second hooking portion from the rear side of the eyeball to the lower side of the lower eyelid.
